# EUROPEAN PATENT APPLICATION

(11) **EP 4 381 941 A1**
(43) Date of publication of application: **12.06.2024**
(21) Application number: 21782577.7
(22) Date of filing: 02.08.2021
(51) Int. Cl.: A01K 5/02, A01K 67/033

(54) **INSECT-FOOD-DOSING MACHINE**

(71) Applicant: Mealfood Europe S.L., 37120 Doñinos de Salamanca (ES)
(72) Inventor: DE DIEGO MATEOS, Sabas, 37120 Doñinos de Salamanca (ES); CASILLAS BARROS, Adriana, 37120 Doñinos de Salamanca (ES); ABORDÁN BERNAL, Ramir, 37120 Doñinos de Salamanca (ES)
(74) Representative: Pons
(86) International application number: PCT/ES2021/070584
(87) International publication number: WO 2023/012379

(57) **Abstract**

Food dispensing machine for insects comprising a hopper (10) having an inlet port (11) for receiving the food and an outlet port (12) for supplying the food, and a dispensing device (20) having an inlet port (21) for receiving the food, which is connected to the outlet port (12) of the hopper (10) and an outlet port (22) for supplying the food in trays. The dispensing device (20) is arranged in a lower position than the hopper (10) and has at least one vibration device (30) to cause the dispensing device (20) to vibrate and dispense the food. The machine also comprises a conveyor (40) arranged in a lower position than the dispensing device (20), said conveyor having an inlet area (41) for loading the trays and an outlet area (42) for unloading the trays full with food. , and being designed to move the trays in a forward movement direction (A) between the inlet area (41) and the outlet area (42) of the conveyor (40).

## Description

### TECHNICAL FIELD

The present invention relates to an insect food dispensing machine.

### PRIOR ART

The current state of animal nutrition, especially in relation to sectors as important as aquaculture or poultry, presents serious problems related to obtaining the proteins necessary to provide a good diet for animals. In this area, an alternative to achieve healthy nutrition is to use insects as a base and/or food supplement. In addition, the use of insects as food brings with it a good number of environmental, health, social and life benefits, which also makes insect-based nutrition an appropriate solution to solve problems related to human nutrition.

Insect farms are known where the breeding, reproduction and fattening of insects takes place. To achieve adequate growth, insects need to have a constant food source during their life cycle, both in the larval stage and in the adult stage.

Generally, insect feeding is done manually. The insects are arranged in trays along with the food, and from time to time the trays are cleaned by removing the manure generated by the insects and providing a new amount of food that is dispensed depending on the population of insects that exists in the tray and their growth stage.

Manual dispensing is unproductive, furthermore, human interaction with insects can cause damage by crushing the insects, or can also cause the transmission of pathogens between insect populations. On the other hand, filling the trays with excess food can cause adult insects to climb on top of the food and escape from the tray. Therefore, systems are required that automate the dispensing of food according to the growth conditions of the insects.

WO2017223096A1 shows an autonomous insect food delivery platform that allows automatic feeding of insects without requiring human interaction. The platform comprises a hopper having an inlet port for receiving the insect food and an outlet port for supplying the insect food, and a dispensing tube having an inlet port for receiving the insect food from the outlet port of the hopper and an outlet port for supplying the insect food on trays. The trays are arranged in a tower according to a vertical distribution, and both the tower with the trays and the hopper with the dispensing tube are arranged on a mobile base that moves the platform through a store where the insects are located. The insects are housed in vertical racks of trays, such that the platform carries the food to a rack and from each tray on the platform the food is supplied to each tray on the rack.

### DESCRIPTION OF THE INVENTION

The object of the invention is to provide an insect food dispensing machine, as defined in the claims.

The invention relates to an insect food dispensing machine comprising a hopper having an inlet port for receiving the insect food and an outlet port for supplying the insect food, and a dispenser having an inlet port, which is connected to the outlet port of the hopper for receiving the insect food and an outlet port for supplying the insect food in trays. The dispenser is arranged in a lower position than the hopper and has at least one vibrator for vibrating the dispenser and dispensing the insect food via the outlet port of the dispenser. The machine additionally comprises a conveyor arranged in a lower position than the dispenser that has an inlet area for loading the trays and an outlet area for unloading the trays filled with the insect food supplied by the dispenser, the conveyor being configured to move the trays according to a direction of advance between the inlet area and the outlet area of the conveyor below the dispenser.

The machine allows for automatic and precise dispensing of food into the trays, since by controlling the vibration force of the vibrator and the advance of the conveyor, the amount of food supplied to the trays can be controlled. Furthermore, the lower arrangement of the dispenser below the hopper, and of the conveyor below the dispenser, allows the force of gravity to be used to facilitate the supply of food from the hopper to the trays, with the food falling into the trays when the vibrator causes the dispenser to vibrate.

In this way, an adequate dispensing of food is achieved that can be adjusted to the needs of the insects depending on their growth phase.

These and other advantages and characteristics of the invention will become evident in view of the figures and the detailed description of the invention.

### DESCRIPTION OF THE DRAWINGS

Figure 1 shows a partial perspective view of an example of the insect food dispensing machine of the invention.
Figure 2 shows a side view of the machine of Figure 1.
Figure 3 shows a front view of the machine of Figure 1.
Figure 4 shows a top plan view of the machine.
Figure 5 shows a perspective view of an example of the insect food dispensing machine with a vibrator on each side wing of the dispenser.

### DETAILED DESCRIPTION OF THE INVENTION

The invention relates to an insect food dispensing machine, particularly food for the tenebrio molitor (commonly called mealworm).

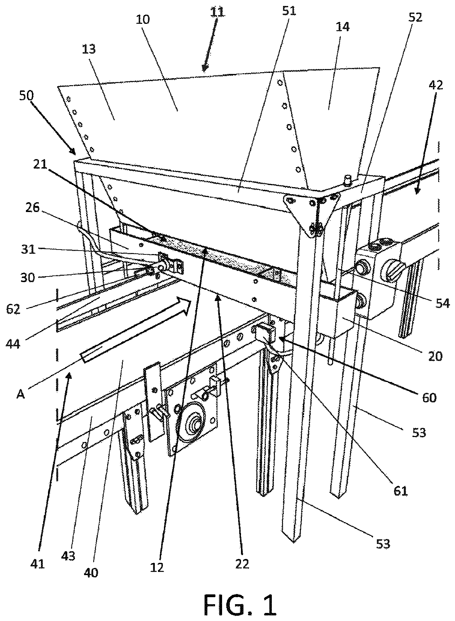

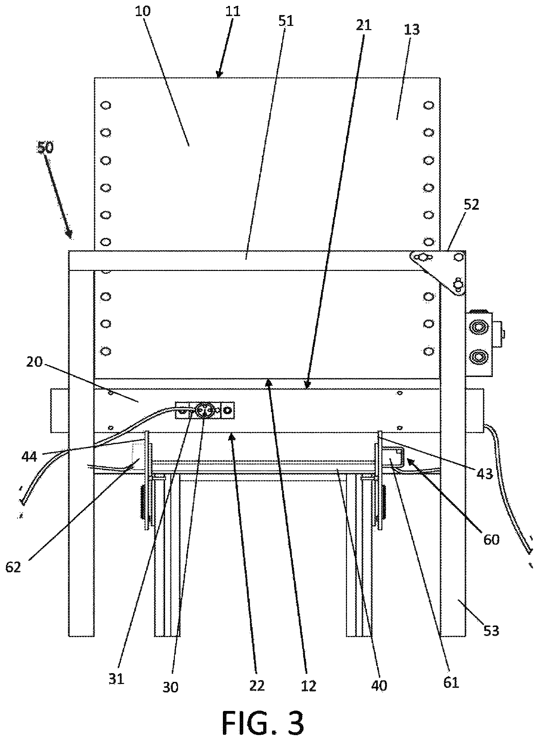

As seen in the example of Figures 1 to 4, the insect food dispensing machine comprises a hopper 10 having an inlet port 11 for receiving the insect food and an outlet port 12 for supplying the insect food, and a dispenser 20 having an inlet port 21, which is connected to the outlet port 12 of the hopper 10 for receiving the insect food and an outlet port 22 for supplying the insect food in trays 1.

The dispenser 20 is arranged in a lower position than the hopper 10 and has at least one vibrator 30 for vibrating the dispenser 20 and dispensing the insect food via the outlet port 22 of the dispenser 20. The force of the vibration allows the amount of food dispensed to be controlled. Thus, the dispenser 20 receives the food from the hopper 10 by gravity, and the vibration provided by the vibrator 30 allows the dispenser 20 to gradually supply the food through the outlet port 22 of the dispenser 20.

The machine additionally comprises a conveyor 40 arranged in a lower position than the dispenser 20 having an inlet area 41 for loading the trays 1 and an outlet area 42 for unloading the trays 1 filled with the insect food supplied by the dispenser 20, the conveyor 40 being configured to move the trays 1 according to a direction of advance A between the inlet area 41 and the outlet area 42 of the conveyor 40 below the dispenser 20. Thus, the conveyor 40 moves the trays 1 to position them below the dispenser 20 from where the food is supplied. Trays 1 may be empty or may contain insects, in a larval stage or in an adult stage.

Preferably, the conveyor 40 of the trays 1 is a conveyor belt, although it may be a chain conveyor or other similar transport element.

Preferably, the dispenser 20 supplies the food in the trays 1 while the conveyor 40 moves the trays 1 in the direction of advance A below the dispenser 20, such that the supply of the food is carried out with the trays 1 in motion.

Preferably the insect food comprises a by-product of a cereal that has been subjected to milling. Milling causes the food to have a particular granulometry that can make dispensing difficult, since the food tends to compact. The dispensing machine of the invention is specially configured to dispense a by-product of a cereal that has been subjected to milling.

Even more preferably, the food comprises bran, which is the product resulting from the milling process from the cereal grain shells that remain after extracting the flour or semolina. For example, the food comprises wheat bran, oat bran, other cereal bran, or bran mixture.

Vibration is especially suitable for supplying a by-product of a cereal that has been subjected to milling, since, in the absence of vibration, that type of food tends to compact and does not fall through the outlet port 22 of the dispenser 20.

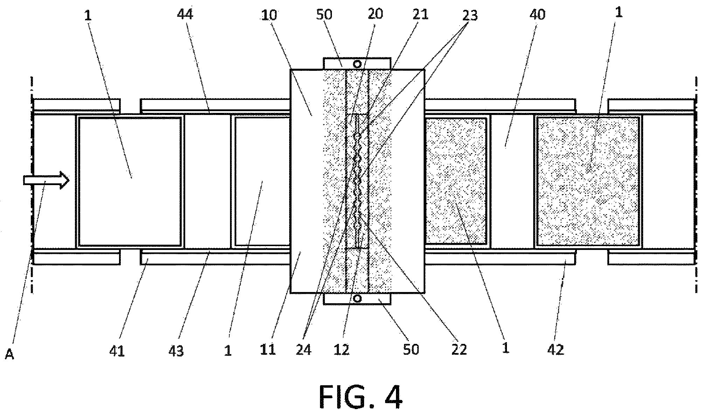

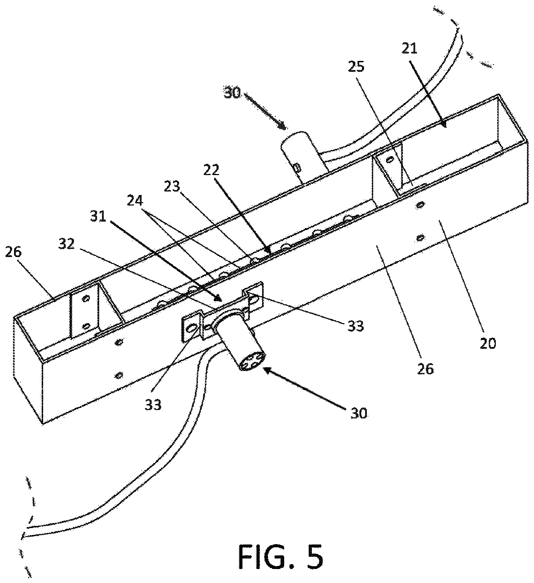

Preferably, as seen in detail in Figures 4 and 5, the outlet port 22 of the dispenser 20 has holes 23 for supplying insect food, the holes 23 being aligned in a direction perpendicular to the direction of advance A of the trays 1. A food curtain having a width similar to the width of the trays 1 is supplied via the holes 23. In this way, as tray 1 advances, a homogeneous distribution of the food in tray 1 is achieved. The diameter of the holes 23 is selected depending on the amount of food that needs to be supplied. The diameter of the holes 23 is also selected depending on the granulometry of the food to be dispensed so that the food only falls when the vibrator 30 is active.

Preferably, the holes 23 are aligned in a single row that extends in the direction perpendicular to the direction of advance A of the trays 1, such that the homogeneous filling of the tray 1 is obtained by supplying the food continuously through the outlet port 22 of the dispenser 20 while the vibrator 30 is active and as the tray 1 advances below the dispenser 20.

As shown in Figures 4 and 5, the holes 23 are equally spaced from each other. In this way, all the holes 23 are spaced at the same distance, which optimizes the homogeneous distribution of the food in the trays 1. Like the diameter of the holes 23, the distance at which the holes 23 are spaced is selected depending on the amount of food that needs to be supplied.

The holes 23 are joined by grooves 24 that transmit the vibration to the holes 23 and that extend in the direction perpendicular to the direction of advance A of the trays 1. When the food is a by-product of a cereal that has been subjected to milling, given the granulometry of that type of food, the holes 23 can become clogged, so the grooves 24 facilitate the dispensing of the food via the holes 23 without requiring an additional vibration force, since the grooves 24 conduct the vibrating waves to the holes 23, undoing the clogging that can occur in the holes 23.

Preferably, the grooves 24 have a thickness less than the diameter of the holes 23. As shown in Figures 4 and 5, the holes 23 are circular holes, and the grooves 24 are straight sections that have a width less than the diameter of the holes 23. This configuration facilitates the food being supplied only via the holes 23 and the grooves 24 only fulfilling the function of transmitting the vibration to the holes 23. Holes 23 and grooves 24 are machined directly into the outlet port 22 of dispenser 20.

The dispenser 20 is attached to the hopper 10 to transmit the vibration of the dispenser 20 to the hopper 10. In this way, the vibrator 30 of the dispenser 20 is used to vibrate the hopper 10 and facilitate the supply of food between the hopper 10 and the dispenser 20.

Preferably the outlet port 12 of the hopper 10 is a rectangular opening. The opening is smaller than the dispenser 20 since the food falls by gravity directly from the outlet port 12 of the hopper 10 to the inlet port 21 of the dispenser 20.

As seen in detail in Figures 1 to 3, the hopper 10 is arranged on a support structure 50 that supports the hopper 10 in an upper position on the conveyor 40, and the dispenser 20 is attached to the support structure 50, leaving the dispenser 20 in an upper position on the conveyor 40, with the inlet port 21 of the dispenser 20 aligned with the outlet port 12 of the hopper 10. The support structure 50 is supported on the ground and keeps the hopper 10 and dispenser 20 cantilevered over the conveyor 40. In this way, when the vibration is activated, the food falls by gravity from the hopper 10 to the dispenser 20, and from the dispenser 20 to the tray 1 that is transferred on the conveyor 40.

The hopper 10 has two oblique faces 13, two straight faces 14 that join the oblique faces 13 to each other, an upper face that corresponds to the inlet port 11, and a lower face that corresponds to the outlet port 12. The two oblique faces 13 gradually reduce the horizontal section of the hopper 10 from the inlet port 11 towards the outlet port 12. The hopper 10 is shaped like a funnel with a rectangular horizontal section.

The support structure 50 has a rectangular upper part with two longitudinal profiles 51 and two transverse profiles 52, and a lower part with four vertical profiles 53 to support the upper part of the support structure 50 on the ground.

As seen in detail in Figures 1 to 3, the support structure 50 has the longitudinal profiles 51 to support the oblique faces 13 of the hopper 10, supporting the longitudinal profiles 51 in an intermediate area of the oblique faces 13. This support area ensures the support of the hopper 10 and at the same time allows the vibration to be transmitted in an intermediate area of the hopper 10, facilitating the transmission of vibration to all points of the hopper 10.

The dispenser 20 is attached to the hopper 10 by means of the support structure 50. The ends of the dispenser 20 are attached by vertical bars 54 to the transverse profiles 52 of the upper part of the support structure 50, and the vibration is transmitted from the dispenser 20 to the hopper 10 via the vertical bars 54 and the support structure 50.

Preferably, the dispenser 20 comprises an elongated sheet having a lower base 25 where the outlet port 22 is located and two side wings 26 projecting from the lower base 25, and where the vibrator 30 is arranged on one of the side wings 26. The dispenser 20 has a substantially rectangular shape and can be a metal plate bent in the shape of a "U", such that being a single piece it facilitates the transmission of vibration.

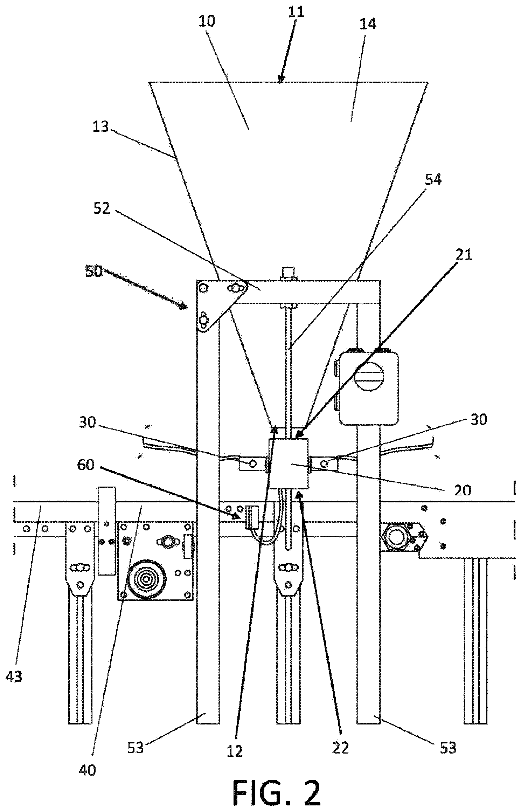

As seen in detail in Figures 2 and 5, each side wing 26 of the dispenser 20 has a vibrator 30. Thus, the dispenser 20 has two vibrators 30 that improve the distribution of the vibration.

Even more preferably, and as seen in the example of Figure 5, the two vibrators 30 are misaligned with each other and are displaced with respect to the centre of the dispenser 20, the vibrator 30 of a side wing 26 is displaced towards one end of the dispenser 20 and the other vibrator 30 of the other side wing 26 is displaced towards the opposite end of the dispenser 20. In this way, a homogeneous distribution of the vibration is obtained, where one vibrator 30 acts from the centre to one end of the dispenser 20, and the other vibrator 30 acts from the centre to the opposite end of the dispenser 20.

Preferably, the vibrator 30 generates a vibration force in a direction perpendicular to the side wing 26. The vibration force is parallel to the direction of advance A of the trays 1. The vibrator 30 generates a forward and backward oscillatory movement in the direction perpendicular to the side wing 26.

Preferably, the vibrator 30 has a spring 31 that transmits the vibration of the vibrator 30 onto the side wing 26 of the dispenser 20, the spring 31 has a base 32 parallel to the side wing 26 and two legs 33 that project perpendicularly from the base 32, the vibrator 30 is connected to the base 32 of the spring 31 to receive the vibration force and the two legs 33 of the spring 31 are attached to the side wing 26 of the dispenser 20 to transmit the vibration force at two points of the side wing 26. The spring 31 has an "II" shape where the base 32 is substantially elongated and tends to buckle when the vibrator 30 acts on the base 32, amplifying the oscillatory movement that is transmitted to the side wing 26 by means of the legs 33 of the spring 31.

The machine additionally comprises a sensor 60 that is arranged downstream of the dispenser 20 according to the direction of advance A to detect the presence of trays 1 on the conveyor 40, the vibrator 30 being configured to vibrate while the sensor 60 detects the presence of a tray 1. When the sensor 60 detects the presence of tray 1, the vibration on the dispenser 20 is activated, and the food begins to be supplied to the tray 1, maintaining the vibration while the sensor 60 is active.

Preferably, the sensor 60 is vertically aligned with the dispenser 20, however, it could be arranged prior to the dispenser 20 and have a delay time to activate the vibration. Preferably, the sensor 60 comprises an emitter 61 that is arranged on a first side of the conveyor 40 to emit a light beam and an optical receiver 62 that is arranged on a second side of the conveyor 40, opposite the first side, to receive the light beam, such that the presence of a tray 1 on the conveyor 40 interrupts the light beam, maintaining the vibration of the vibrator 30 as long as the receiver 62 of the sensor 60 does not receive the light beam.

The conveyor 40 has guides 43 and 44 on its sides to guide the trays 1 on the conveyor 40. The emitter 61 is arranged in the guide 43 on the first side and the receiver 62 is arranged in the guide 44 on the second side. As seen in Figure 1, the guides 43 and 44 have holes to allow the light beam to pass between the emitter 61 and the receiver 62.

## Claims

1. An insect food dispensing machine comprising a hopper (10) having an inlet port (11) for receiving the insect food and an outlet port (12) for supplying the insect food, and a dispenser (20) having an inlet port (21) connected to the outlet port (12) of the hopper (10) for receiving the insect food and an outlet port (22) for supplying the insect food in trays (1), **characterized in that** the dispenser (20) is arranged in a lower position than the hopper (10) and has at least one vibrator (30) to vibrate the dispenser (20) and dispense the insect food via the outlet port (22) of the dispenser (20), and **in that** the machine additionally comprises a conveyor (40) arranged in a lower position than the dispenser (20) that has an inlet area (41) for loading the trays (1) and an outlet area (42) for unloading the trays (1) filled with the insect food supplied by the dispenser (20), the conveyor (40) being configured to move the trays (1) according to a direction of advance (A) between the inlet area (41) and the outlet area (42) of the conveyor (40) below the dispenser (20).

2. The machine according to claim 1, wherein the outlet port (22) of the dispenser (20) has holes (23) for supplying insect food, the holes (23) being aligned in a direction perpendicular to the direction of advance (A) of the trays (1).

3. The machine according to claim 2, wherein the holes (23) are equally spaced from each other.

4. The machine according to claim 2 or 3, wherein the holes (23) are joined by grooves (24) that transmit the vibration to the holes (23) and that extend in the direction perpendicular to the direction of advance (A) of the trays (1).

5. The machine according to the preceding claim, wherein the grooves (24) have a thickness less than the diameter of the holes (23).

6. The machine according to any of the preceding claims, wherein the dispenser (20) is attached to the hopper (10) to transmit the vibration of the dispenser (20) to the hopper (10).

7. The machine according to the preceding claim, wherein the hopper (10) is arranged on a support structure (50) that supports the hopper (10) in an upper position on the conveyor (40), and the dispenser (20) is attached to the support structure (50), leaving the dispenser (20) in an upper position on the conveyor (40), with the inlet port (21) of the dispenser (20) aligned with the outlet port (12) of the hopper (10).

8. The machine according to the preceding claim, wherein the hopper (10) has two oblique faces (13) that gradually reduce the horizontal section of the hopper (10) from the inlet port (11) towards the outlet port (12), and the support structure (50) has longitudinal profiles (51) to support the oblique faces (13) of the hopper (10), supporting the longitudinal profiles (51) in an intermediate area of the oblique faces (13).

9. The machine according to any of the preceding claims, wherein the dispenser (20) comprises an elongated sheet having a lower base (25) where the outlet port (22) is located and two side wings (26) projecting from the lower base (25), and wherein the vibrator (30) is arranged in one of the side wings (26).

10. The machine according to the preceding claim, wherein each side wing (26) of the dispenser (20) has a vibrator (30).

11. The machine according to claim 10, wherein the two vibrators (30) are misaligned with each other and are displaced with respect to the centre of the dispenser (20), the vibrator (30) of a side wing (26) is displaced towards one end of the dispenser (20) and the other vibrator (30) of the other side wing (26) is displaced towards the opposite end of the dispenser (20).

12. The machine according to any of claims 9 to 11, wherein the vibrator (30) generates a vibration force in a direction perpendicular to the side wing (26).

13. The machine according to the preceding claim, wherein the vibrator (30) has a spring (31) that transmits the vibration of the vibrator (30) onto the side wing (26) of the dispenser (20), the spring (31) has a base (32) parallel to the side wing (26) and two legs (33) that project perpendicularly from the base (32), the vibrator (30) is connected to the base (32) of the spring (31) to receive the vibration force and the two legs (33) of the spring (31) are attached to the side wing (26) of the dispenser (20) to transmit the vibration force at two points of the side wing (26).

14. The machine according to any of the preceding claims, which additionally comprises a sensor (60) that is arranged downstream of the dispenser (20) according to the direction of advance (A) to detect the presence of trays (1) on the conveyor (40), the vibrator (30) being configured to vibrate while the sensor (60) detects the presence of a tray (1).

15. The machine according to the preceding claim, wherein the sensor (60) comprises an emitter (61) that is arranged on a first side of the conveyor (40) to emit a light beam and an optical receiver (62) that is arranged on a second side of the conveyor (40), opposite to the first side, to receive the light beam, such that the presence of a tray (1) on the conveyor (40) interrupts the light beam, maintaining the vibration of the vibrator (30) while the receiver (62) of the sensor (60) does not receive the light beam.
